# EUROPÄISCHE PATENTSCHRIFT

(11) **EP 3 344 663 B1**
(45) Veröffentlichungstag und Bekanntmachung des Hinweises auf die Patenterteilung: **19.02.2020**
(21) Anmeldenummer: 16756968.0
(22) Anmeldetag: 10.08.2016
(51) Int. Cl.: C08F 2/22, C08F 18/00, C08F 20/00, C08F 10/00, C08F 12/00, A01N 43/56

(54) **PENFLUFENHALTIGE POLYMERPARTIKEL**
POLYMER PARTICLE CONTAINING PENFLUFEN
PARTICULE POLYMERE CONTENANT DU PENFLUFEN

(30) Priorität: 02.09.2015 EP 15183408
(43) Veröffentlichungstag der Anmeldung: 11.07.2018
(73) Patentinhaber: LANXESS Deutschland GmbH, 50569 Köln (DE)
(72) Erfinder: UHR, Hermann, 51373 Leverkusen (DE); JAETSCH, Thomas, 50933 Köln (DE)
(86) Internationale Anmeldenummer: PCT/EP2016/069047
(87) Internationale Veröffentlichungsnummer: WO 2017/036755

(56) Entgegenhaltungen:
- EP-A1- 2 443 927

## Beschreibung

Die Erfindung betrifft penflufenhaltige Polymerpartikel, diese enthaltende penflufenhaltige Mittel, Verfahren zu deren Herstellung und deren Verwendung zum Schutz von technischen Materialien, insbesondere von Holz, Holzprodukten und Holz-Plastik-Kompositen.

Penflufen (N-(2-[1,3-Dimethylbuthylphenyl]-5-fluor-1,3-dimethyl-1H-pyrazol-4-carboxamid, CAS Nr. 494793-67-8) ist ein fungizider Wirkstoff aus der Klasse der Pyrazolylcarboxanilide der Formel (I) und aus der WO 20031010149 A1 bekannt.

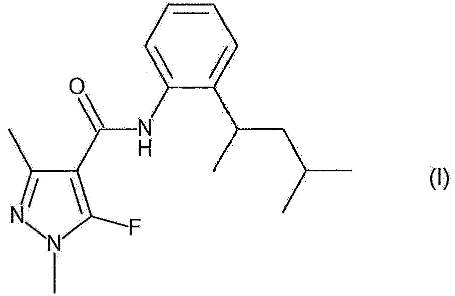

Aus der WO 2012055673 A1 und der WO 2012055674 A1 ist bekannt, dass Penflufen insbesondere zur Bekämpfung von holzzerstörende Pilzen genutzt werden kann.

Die Verkapselung fungizider Wirkstoffe kann aus verschiedenen Gründen sinnvoll sein. Neben der Möglichkeit, dosiert Wirkstoffe freizusetzen und damit den Langzeitschutz der Fungizide zu verlängern, kann auch die Reduktion an organischen Lösungsmitteln oder Emulgatoren ein Grund sein, wie aus der EP 1742531 B1 bekannt ist.

Die EP 1742531 B1 beschreibt ein Verfahren zur Herstellung verkapselter wasserunlöslicher Fungizide, bei dem über eine klassische radikalische Emulsionspolymerisation, die Fungizide in den Monomeren gelöst und dann die Monomere polymerisiert werden. Bei der klassischen Emulsionspolymerisation werden Wasser, Monomer oder Monomermischungen, gegebenenfalls Tenside, Schutzkolloide und ein wasserlöslicher Initiator emulgiert und diese Emulsion polymerisiert. Hierbei findet die Polymerisation in der Wasserphase oder in Micellen des Tensids statt. Das Monomer wird über die Wasserphase aus den Öltröpfen kontinuierlich für die Polymerisation nachgeliefert. Nachteilig an dem aus der EP 1742531 B1 bekannten Verfahren ist, dass Penflufen nach diesem Verfahren nicht verkapselt werden kann, da eine zu große Menge an kristallisiertem Penflufen oder mit dem Polymeren verklebtes Penflufen entsteht.

Die Verwendung von Mischungen aus Penflufen und weiteren Wirkstoffen im Material- und Holzschutz ist aus der EP-A-2443927 bekannt.

Es bestand daher weiterhin Bedarf nach stabilen, penflufenhaltigen Polymerpartikeln.

Überraschend wurde jetzt gefunden, dass stabile, penflufenhaltige Polymerpartikel hergestellt werden können, wenn öllösliche radikalische Initiatoren bei der Emulsionspolymerisation eingesetzt werden.

Gegenstand der Erfindung sind daher penflufenhaltige Polymerpartikel, die durch Polymerisation mindestens eines ethylenisch, ungesättigten Monomeren in Gegenwart von Penflufen oder dessen Salze und Säureadditionsverbindungen und in Gegenwart eines öllöslichen radikalischen Initiators in einer Öl-in-Wasser Emulsionspolymerisation hergestellt werden, dadurch gekennzeichnet, dass als ein ethylenisch ungesättigter Monomer ein neutraler, ungeladener, ethylenisch ungesättigter Monomer des Typs M1 ausgewählt aus der Gruppe Styrol, Divinylbenzol, Ester monoethylenisch ungesättigter Mono- und Dicarbonsäuren mit 3 bis 8 C-Atomen und insbesondere 3 oder 4 C-Atomen mit C₁-C₂₀-Alkanolen oder mit C₅-C₈-Cycloalkanolen in einer Menge von mindestens 60 Gew. %, bezogen auf die Gesamtmenge der eingesetzten Monomeren, eingesetzt wird..

Wenn in der Anmeldung auf penflufenhaltige Polymerpartikel bezug genommen wird, sind dies Polymerpartikel enthaltend Penflufen oder dessen Salze und Säureadditionsverbindungen.

Penflufen kann erfindungsgemäß sowohl als Racemat, in enantiomerenreiner Form oder als angereichertes Enantiomerengemisch eingesetzt werden. Auch eine Verwendung als Salz oder Säureadditionsverbindung ist möglich, wobei unter Salzen insbesondere Natrium-, Kalium-, Magnesium-, Calcium-, Zink-, Aluminium-, Eisen- und Kupfer-Salze verstanden wird und unter Säureadditionsverbindungen insbesondere Addukte mit Halogenwasserstoffsäuren, z.B. Chlorwasserstoff und Bromwasserstoff, Carbonsäuren, wie z.B. Ameisensäure, Essigsäure, Weinsäure und Oxalsäure, Sulfonsäuren, wie z.B. p-Toluolsulfonsäure sowie Schwefelsäure, Phosphorsäure und Salpetersäure verstanden wird. Bevorzugt wird Penflufen eingesetzt.

Die ethylenisch, ungesätigten Monomeren können entweder als Reinstoffe oder als Mischungen aus mehreren ethylenisch, ungesätigten Monomeren bestehen.

Bevorzugt handelt es sich um neutrale, ungeladene, ethylenisch ungesätigten Monomere, die die Bezeichnung M1 erhalten.

Geeignete Monomere M1 umfassen bevorzugt vinylaromatische Monomere wie Styrol, Divinylbenzol, Ester monoethylenisch ungesättigter Mono- und Dicarbonsäuren mit 3 bis 8 und insbesondere 3 oder 4 C-Atomen mit C₁-C₂₀-Alkanolen oder mit C₅-C₈-Cyclo-alkanolen, insbesondere die Ester der Acrylsäure, der Methacrylsäure, der Crotonsäure, die Diester der Maleinsäure, der Fumarsäure und der Itaconsäure und besonders bevorzugt die Ester der Acrylsäure mit C₁-C₁₈-Alkanolen (= C₁-C₁₈-Alkylacrylate) wie Methylacrylat, Ethylacrylat, n-Butylacrylat, Isobutylacrylat, tert.-Butylacrylat, n-Hexylacrylat, 2-Ethylhexylacrylat, 3-Propylheptylacrylat und Stearylacrylat sowie die Ester der Methacrylsäure mit C₁-C₁₈-Alkanolen wie Methylmethacrylat, Ethylmethacrylat, n-Butylmethacrylat, Isobutylmethacrylat, tert.-Butylmethacrylat, n-Hexylmethacrylat und Stearylmethacrylat. Geeignete Monomere M1 sind außerdem Vinyl- und Allylester aliphatischer Carbonsäuren mit 1 bis 20 C-Atomen, beispielsweise Vinylacetat, Vinylpropionat sowie die Vinylester der Versatic®-Säuren (Vinylversatate), Vinylhalogenide wie Vinylchlorid und Vinylidenchlorid, konjugierte Diolefine wie Butadien und Isopren, sowie C₂-C₆-Olefine, wie Ethylen, Propen, 1-Buten und n-Hexen. Bevorzugte Monomere sind vinylaromatische Monomere, insbesondere Styrol, Divinylbenzol, C₁-C₂₀-Alkylacrylate, insbesondere C₁-C₁₈-Alkylacrylate und C₁-C₁₈-Alkylmethacrylate.

Des Weiteren eignen sich als Monomere M1 auch Amide der vorgenannten ethylenisch ungesättigten Carbonsäuren, insbesondere Acrylamid und Methacrylamid, ethylenisch ungesättigte Nitrile wie Methacrylnitril und Acrylnitril, Hydroxyalkylester der vorgenannten α,β-ethylenisch ungesättigten C₃-C₈-Monocarbonsäuren und der C₄-C₈-Dicarbonsäuren insbesondere Hydroxyethylacrylat, Hydroxyethylmethacrylat, 2- und 3-Hydroxypropylacrylat, 2- und 3-Hydroxypropylmethacrylat, Ester der vorstehend genannten monoethylenisch ungesättigten Mono-und Dicarbonsäuren mit C₂-C₄-Polyalkylenglykolen, insbesondere die Ester dieser Carbonsäuren mit Polyethylenglykol oder Alkyl-Polyethylenglykolen, wobei der (Alkyl)polyethylenglykol-Rest üblicherweise ein Molekulargewicht im Bereich von 100 bis 3000 g/mol aufweist.

Weitere Monomere M1 sind N-Vinylamide wie N-Vinylformamid, N-Vinylpyrrolidon, N-Vinylimidazol und N-Vinylcaprolactam.

Das Polymer baut sich aus mindestens 60 Gew. % Monomeren M1 auf, bezogen auf die Gesamtmenge der eingesetzten Monomeren. Bevorzugt besteht der Anteil an Monomeren M1 mindestens 70 Gew. %, besonders bevorzugt mindestens 80 Gew. %.

Zusätzlich zu den Monomeren M1 können gegebenenfalls auch ethylenisch, ungesätigte Monomere mit anionischen oder kationischen Gruppen, also geladene, ethylenisch, ungesättigte Monomere mit einpolymerisiert werden. Die ethylenisch, ungesättigten Monomeren mit anionischen oder kationischen Gruppen erhalten die Bezeichnung M2.

Zu den Monomeren M2 zählen insbesondere monoethylenisch ungesättigte Monomere, die wenigstens eine anionische Gruppe aufweisen, insbesondere Monomere, die wenigstens eine Säuregruppe, vorzugsweise wenigstens eine Sulfonsäuregruppe, eine Phosphonsäuregruppe oder ein oder zwei Carbonsäuregruppen aufweisen, sowie die Salze der Monomeren insbesondere die Alkalimetallsalze, z. B. die Natrium- oder Kaliumsalze sowie die Ammoniumsalze. Des Weiteren ethylenisch ungesättigte Sulfonsäuren insbesondere Vinylsulfonsäure, 2-Acrylamido-2-methylpropansulfonsäure, 2-Acryloxyethansulfonsäure und 2-Methacryloxyethansulfonsäure, 3-Acryloxy- und 3-Methacryloxypropansulfonsäure, Vinylbenzolsulfonsäure und deren Salze, ethylenisch ungesättigte Phosphonsäuren wie Vinylphosphonsäure und Vinylphosphonsäuredimethylester und deren Salze und α,β-ethylenisch ungesättigte C₃-C₈-Mono-und C₄-C₈-Dicarbonsäuren insbesondere Acrylsäure, Methacrylsäure, Crotonsäure, Maleinsäure, Fumarsäure und Itaconsäure. Bevorzugt werden als Monomere M2 α,β-ethylenisch ungesättigte C₃-C₈-Mono- und C4-C8-Dicarbonsäuren eingesetzt.

Zu den Monomeren M2 können weiterhin insbesondere monoethylenisch ungesättigte Monomere zählen, die wenigstens eine kationische Gruppe und/oder wenigstens eine im wässrigen protonierbare Gruppe aufweisen. Zu den kationischen Monomeren M2 zählen insbesondere solche, die eine protonierbare Aminogruppe, eine quaternäre Ammoniumgruppe, eine protonierbare Iminogruppe oder eine quaternisierte Iminogruppe aufweisen. Beispiele für Monomere M2 mit einer protonierbaren Iminogruppe sind N-Vinylimidazol und Vinylpyridine. Beispiele für Monomere M2 mit einer quaternisierten Iminogruppe sind N-Alkylvinylpyridiniumsalze und N-Alkyl-N'-vinylimidazoliniumsalze wie N-Methyl-N'-vinylimidazoliniumchlorid oder Methosulfat.

Beispiele für Monomere M2 mit protonierbaren Aminogruppen sind beispielsweise und vorzugsweise 2-(N,N-Dimethylamino)ethylacrylat, 2-(N,N-Dimethylamino)ethylmethacrylat, 2-(N,N-Dimethylamino)ethylacrylamid, 3-(N,N-Dimethylamino)propyl-acrylamid, 3-(N,N-Dimethylamino)propylmethacrylamid, 2-(N,N-Dimethylamino) ethylmethacrylamid, sie können beispielsweise vorliegen als Cl⁻, HSO⁴⁻, 1/2 SO₄²- oder CH₃OSO₃⁻-Salze. Monomere mit quartären Ammoniumverbindungen sind beispielsweise und vorzugsweise 2-(N,N,N-Trimethylammonium)ethylacrylat-Chlorid, 2-(N,N,N-Trimethylammonium)ethylmethacrylat-Chlorld, 2-(N,N,N-Trimethylammonium)-ethylmethacrylamid-Chlorid, 3-(N,N,N-Trimethylammonium)propylacrylamid-Chlorid, 3-(N,N,N-Trimethylammonium) propylmethacrylamid-Chlorid, 2-(N,N,N-Trimethylammonium)-ethylacrylamid-Chlorid, sowie die entsprechenden Methosulfate und Sulfate.

Der Anteil der Monomere M2 ist vorzugsweise nicht größer als 35 Gew.-%, besonders bevorzugt nicht größer als 20 Gew.-%, bezogen auf die Gesamtmenge der eingesetzten Monomeren. Ganz besonders bevorzugt wird 80 Gew. % bis 99.9 Gew. % Monomere M1 und 0,1 Gew.% bis 20 Gew. % Monomere M2, bezogen auf die Gesamtmenge der eingesetzten Monomeren, eingesetzt.

In einer weiteren bevorzugten Ausführungsform ist die Menge an geladenen Monomeren M2 im Polymer kleiner als 0,01 Gew. % bezogen auf die Gesamtmenge an eingesetzten Monomeren.

Als radikalischer, öllöslicher Initiator können öllösliche Per-, Oxo- und Azoverbindungen, verwendet werden, die einzeln oder auch als Gemische eingesetzt werden können. Hierzu zählen Azoverbindungen, wie 2,2'-Azobis-isobutyronitril, 2,2'-Azobis(2-methylbutyronitril), 2,2'Azobis[2-methyl-N-(-2-hydroxyethyl)propionamid, 1,1'-Azobis(1-cyclohexancarbonitril), 2,2'-Azobis(2,4-dimethylvaleronitril), 2,2'-Azobis(N,N'-dimethylen-isobutyroamidin)dihydrochlorid, und 2,2'-Azobis(2-amidinopropan)dihydrochlorid, organische oder anorganische Peroxide wie Diacetylperoxid, Di-tert.-butylperoxid, Diamylperoxid, Dioctano-ylperoxid, Didecanoylperoxid, Dilauroylperoxid, Dibenzoylperoxid, Bis(o-toluyl)peroxid, Succinylperoxid, tert.-Butylperacetat, tert.-Butylpermaleinat, tert.-Butylperisobutyrat, tert.-Butylperpivalat, tert.-Butylperoctoat, tert.-Butylperneodecanoat, tert.-Butylperbenzoat, tert.-Butylperoxid, tert.-Butylhydroperoxid, Cumolhydroperoxid, tert.-Butylperoxi-2-ethylhexanoat und Diisopropylperoxidicarbamat.

Ganz besonders bevorzugt handelt es sich bei den radikalischen Initiatoren um 2,2'-Azobis-isobutyronitril, 2,2'-Azobis(2-methylbutyronitril) oder Gemische dieser Initiatoren. Die radikalischen Initiatoren werden im Allgemeinen in einer Menge von 0,1 Gew. % bis 5 Gew. %, bezogen auf die Gesamtmenge Monomeren eingesetzt. Bevorzugt wird der radikalische Initiator in einer Menge von 0,1 Gew. % bis 3 Gew. %, bezogen auf die Gesamtmenge der Monomeren eingesetzt.

Zudem können gegebenenfalls weitere Hilfsstoffe wie z.B. Costabilisatoren, oberflächenaktive Substanzen, wie z.B. Schutzkolloide und niedermolekulare Emulgatoren, Lösungsmittel und Weichmacher eingesetzt werden. Falls Hilfsstoffe eingesetzt werden, sind dies bevorzugt Costabilisatoren, Emulgatoren und Lösungsmittel.

Die gegebenenfalls eingesetzten Costabilisatoren sind Stoffe, die die Mikroemulsion gegen Ostwaldreifung stabilisieren und eine sehr geringe Löslichkeit in der kontinuierlichen Phase aufweisen. Bevorzugt handelt es sich um langkettige, lineare, cyklische oder verzweigte, aliphatische Kohlenwasserstoffe ausgewählt aus der Gruppe C₁₄-C₂₅-Alkane und C₁₄-C₂₅-Cycloalkane. Besonders bevorzugt handelt es sich um C₁₄-C₁₈-Alkane und C₁₄-C₁₈-Cycloalkane, wie ganz besonders bevorzugt um Tetradecan, Pentadecan, Hexadecan, Heptadecan, Octadecan und Nonadecan oder Gemische aus diesen Costabilisatoren.

Wenn Costabilisatoren eingesetzt werden, dann liegt die Menge im Allgemeinen zwischen 0,05 bis 20 Gew.%, bevorzugt zwischen 0,1 bis 15 Gew.% und ganz besonders bevorzugt zwischen 0,2 und 10 Gew. % bezogen auf die Gesamtmenge der eingesetzten Monomeren.

Als Lösungsmittel und Weichmacher können im Allgemeinen alle Stoffe eingesetzt werden, die sich gegenüber den Monomeren, dem entstandenen Polymeren und dem Penflufen inert verhalten.

Als bevorzugte Lösungsmittel werden inerte, dipolare, aprotische, organische Lösungsmittel, wie insbesondere Ester zweiwertiger Carbonsäuren, beispielsweise und vorzugsweise Mischungen enthaltend Diisobutyladipat, Diisobutylglutarat, Diisobutylsuccinat (z.B. Rhodiasolv DIB), wie auch Benzylalkohol, Polyethylenglycole, Polypropylenglycole, eingesetzt.

Als Weichmacher werden bevorzugt Phthalate, wie insbesondere Diethylhexylphthalat (DEHP), Dibutylphthalat (BBP), Diisononylphthalat (DINP), Diisodecylphthalat (DIDP), Diisooctylphthalat (DNOP), Diisobutylphthalat (DIBP), Diisohexylphthalat, Diisoheptylphthalat, Di-n-octylphthalat, Diisoundecylphthalat, Diisotredecylphthalate; Adipate, wie insbesondere Diethylhexyladipat (DEHA), Diisooctyladipat, Diisononyladipat, Polyester der Adipinsäure oder Glutarsäure, wie insbesondere Ultramoll IV® der Lanxess Deutschland GmbH; Trialkylester der Zitronensäure oder acetylierte Trialkylester der Zitronensäure, wie insbesondere Acetyltributylcitrat (ATBC); Ester der Trimellitsäure, wie insbesondere Tri(2-ethylhexyl)trimellitat, Tri(isooctyl)trimellitat, Triisononyl)trimellitat; 1,2-Dicyclohexylbasierte Weichmacher, wie insbesondere 1,2-Cyclohexandicarbonsäurenonylester (Hexamoll®, DINCH); Alkylsulfonsäureester des Phenols, wie insbesondere Mesamoll® der Lanxess Deutschland GmbH (CAS-Nr 091082-17-6); Acetylierte Mono- und Diglyceride; Benzoesäurediester, wie insbesondere Dialkylenglycolen, wie insbesondere Dipropylenglycoldibenzoat oder Isononylbenzoat; Trimethylolpropanester wie insbesondere Trimethylolpropan-benzoat-2-ethylhexanoat-Gemische; Dialkylester der Terephthalsäure, wie insbesondere Di-2-ethylhexyl-terephthalat, eingesetzt.

Als oberflächenaktive Substanzen zählen sowohl Schutzkolloide als auch niedermolekulare Emulgatoren, wobei letztere im Unterschied zu den Schutzkolloiden vorzugsweise ein Molekulargewicht unterhalb von 2000 g/mol, insbesondere unterhalb 1000 g/mol (Massenmittel) aufweisen. Die Schutzkolloide und niedermolekularen Emulgatoren können sowohl kationischer, anionischer, neutraler als auch zwitterionischer Natur sein.

Beispiele für anionische oberflächenaktive Substanzen sind anionische niedermolekulare Emulgatoren wie beispielsweise und vorzugsweise Alkylphenylsulfonate, Phenylsulfonate, Alkylsulfate, Alkylsulfonate, Alkylethersulfate, Alkylphenolethersulfate, Alkylpolyglykoletherphosphate, Alkyldiphenylethersulfonate, Polyarylphenyletherphosphate, Alkylsulfosuccinate, Olefinsulfonate, Paraffinsulfonate, Petroleumsulfonate, Tauride, Sarkoside, Fettsäuren, Alkylnaphthalinsulfonsäuren, Naphthalinsulfonsäuren, einschliesslich ihrer Alkali-, Erdalkali-, Ammonium- und Amin-Salze.

Anionische Schutzkolloide sind beispielsweise und vorzugsweise Ligninsulfonsäuren, Kondensationsprodukte sulfonierter Naphthaline mit Formaldehyd oder mit Formaldehyd und Phenol und gegebenenfalls Harnstoff sowie Kondensationsprodukte aus Phenolsulfonsäure, Formaldehyd und Harnstoff, Lignin-Sulfit-Ablauge und Ligninsulfonate sowie Polycarboxylate wie Polyacrylate, Maleinsäureanhydrid/Olefin-Copolymere sowie die Alkali-, Erdalkali-, Ammonium- und Amin-Salze der vorgenannten Schutzkolloide. Nichtionische Schutzkolloide sind beispielsweise und vorzugsweise Polyethylenglykol, Polypropylenglykol, Polyethylenglykolpolypropylenglykol-Blockcopolymere, Polyethylenglykolalkylether, Polypropylenglykolalkylether, Polyethylenglykolpolypropylen-glykolether-Blockcopolymere und deren Gemische.

Weitere, höhermolekulare Schutzkolloide, sind beispielsweise und vorzugsweise Carboxymethylcellulose, natürliche und synthetische pulvrige, körnige oder latexförmige Polymere, wie Gummi Arabicum, Polyvinylalkohol, Polyvinylacetat, sowie natürliche Phospholipide, wie Kephaline und Lecithine und synthetische Phospholipid sowie Paraffinöle.

Nichtionische niedermolekulare Emulgatoren sind beispielsweise und vorzugsweise Alkylphenolalkoxylate, Alkoholalkoxylate, Fettaminalkoxylate, Polyoxyeethylenglycerolfettsäureester, Rizinusölalkoxylate, Fettsäurealkoxylate, Fettsäureamidalkoxylate, Fettsäurepolydiethanolamide, Lanolinethoxylate, Fettsäurepolyglykolester, Isotridecylalkohol, Fettsäureamide, Methylcellulose, Fettsäureester, Silicon-Öle, Alkylpolyglykoside und Glycerolfettsäureester.

Kationische niedermolekulare Emulgatoren sind beispielsweise und vorzugsweise quartäre Ammoniumsalze, z. B. Trimethyl- und Triethyl-C₆-C₃₀-alkylammoniumsalze, wie Cocotrimethylammoniumsalze, Trimethylcetylammoniumsalze, Dimethyl- und Diethyl-di-C₄-C₂₀-alkylammoniumsalze, wie Didecyldimethylammoniumsalze und Dicocodimethylammoniumsalze, Methyl- und Ethyl-tri-C₄-C₂₀-alkylammoniumsalze, wie Methyltrioctylammoniumsalze, C₁-C₂₀-Alkyl-di-C1-C4-alkylbenzylammoniumsalze, wie Triethylbenzylammoniumsalze und Cocobenzyldimethylammoniumsalze, Methyl- und Ethyl-di-C₄-C₂₀-alkylpoly(oxyethyl)ammoniumsalze, z. B. Didecylmethylpoly(oxyethyl) ammoniumsalze, N-C₆-C₂₀-Alkylpyridiniumsalze, z. B. N-Laurylpyridiniumsalze, N-Methyl- und N-Ethyl-N-C₆-C₂₀-alkylmorpholiniumsalze, sowie N-Methyl- und N-Ethyl-N'-C₆-C₂₀-alkylimidazoliniumsalze, insbesondere die Halogenide, Borate, Carbonate, Formiate, Acetate, Propionate, Hydrogencarbonate, Sulfate und Methosulfate.

Zwitterionische niedermolekulare Emulgatoren sind solche mit betainischen Strukturen. Derartige Substanzen sind dem Fachmann bekannt und können einschlägigem Stand der Technik entnommen werden (siehe beispielsweise R.Heusch, in Ullmanns Encyclopedia of Industrial Chemistry, 5th. Ed. On CD-ROM, Wiley-VCH 1997, "Emulsions", Kapitel 7, Tabelle 4).

Die Menge an niedermolekularem Emulgator liegt üblicherweise im Bereich von 0,1 Gew. % bis 15 Gew. %, insbesondere im Bereich von 0,2 Gew. % bis 12 Gew. % und besonders bevorzugt 0,7 Gew. % bis 10 Gew. % bezogen auf die Gesamtmenge der eingesetzten Monomeren.

Zur Erhöhung der Wirksamkeit, aber auch zur Konservierung der entstandenen Suspensionen, können die penflufenhaltigen Polymerpartikel gegebenenfalls weitere antimikrobiell wirksame Verbindungen, Fungizide, Bakterizide, Herbizide, Insektizide oder andere Wirkstoffe enthalten. Diese Mischungen können ein noch breiteres Wirkungsspektrum besitzen. Diese Verbindungen werden wie das Penflufen in den Monomeren gelöst oder dispergiert eingesetzt. In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen:
Triazole wie:
   Azaconazol, Azocyclotin, Bitertanol, Bromuconazol, Cyproconazol, Diclobutrazol, Difenoconazol, Diniconazol, Epoxyconazol, Etaconazol, Fenbuconazol, Fenchlorazol, Fenethanil, Fluquinconazol, Flusilazol, Flutriafol, Furconazol, Hexaconazol, Imibenconazol, Ipconazol, Isozofos, Myclobutanil, Metconazol, Paclobutrazol, Penconazol, Propioconazol, Prothioconazol, Simeoconazol, (+)-cis-1-(4-chlorphenyl)-2-(1H-1,2,4-triazol-1-yl)-cycloheptanol, 2-(1-tert-Butyl)-1-(2-chlorphenyl)-3-(1,2,4-triazol-1-yl)-propan-2-ol, Tebuconazol, Tetraconazol, Triadimefon, Triadimenol, Triapenthenol, Triflumizol, Triticonazol, Uniconazol sowie deren Metallsalze und Säureaddukte;
Imidazole wie:
   Clotrimazol, Bifonazol, Climbazol, Econazol, Fenapamil, Imazalil, Isoconazol, Ketoconazol, Lombazol, Miconazol, Pefurazoat, Prochloraz, Triflumizol, Thiazolcar 1-Imidazolyl-1-(4'-chlorophenoxy)-3,3-dimethylbutan-2-on sowie deren Metallsalze und Säureaddukte;
Pyridine und Pyrimidine wie:
   Ancymidol, Buthiobat, Fenarimol, Mepanipyrin, Nuarimol, Pyroxyfur, Triamirol;
Succinat-Dehydrogenase Inhibitoren wie:
   Benodanil, Carboxim, Carboximsulfoxid, Cyclafluramid, Fenfuram, Flutanil, Furcarbanil, Furmecyclox, Mebenil, Mepronil, Methfuroxam, Metsulfovax, Nicobifen, Pyro¬carbolid, Oxycarboxin, Shirlan, Seedvax;
Naphthalin-Derivate wie:
   Terbinafin, Naftifin, Butenafin, 3-Chloro-7-(2-aza-2,7,7-trimethyl-oct-3-en-5-in);
Sulfenamide wie:
   Dichlofluanid, Tolylfluanid, Folpet, Fluorfolpet; Captan, Captofol;
Benzimidazole wie:
   Carbendazim, Benomyl, Fuberidazole, Thiabendazol oder deren Salze;
Morpholinderivate wie:
   Aldimorph, Dimethomorph, Dodemorph, Falimorph, Fenpropidin, Fenpropimorph, Tridemorph, Trimorphamid und ihre arylsulfonsauren Salze, wie z.B. p-Toluolsulfonsäure und p-Dodecylphenylsulfonsäure;
Benzthiazole wie:
   2-Mercaptobenzothiazol;
Benzthiophendioxide wie:
   Benzo[b]thiophen-S,S-dioxid-carbonsäurecyclohexylamid;
Benzamide wie:
   2,6-Dichloro-N-(4-trifluoromethylbenzyl)-benzamid, Tecloftalam;
Borverbindungen wie:
   Borsäure, Borsäureester, Borax;
Formaldehyd und Formaldehydabspaltende Verbindungen wie:
   Benzylalkoholmono-(poly)-hemiformal, 1,3-Bis(hydroxymethyl)-5,5-dimethylimidazolidine-2,4-dione (DMDMH), Bisoxazolidine, n-Butanol-hemiformal, Cis 1-(3-Chlorallyl)-3,5,7-triaza-1-azoniaadamantane chlorid, 1-[1,3-Bis(hydroxymethyl-2,5-dioxoimidazolidin-4-yl]-1,3-bis(hydroxymethyl)urea, Dazomet, Dimethylolharnstoff, 4,4-Dimethyl-oxazolidine, Ethylen¬glycol-hemiformal, 7-Ethylbicyclooxazolidine, Hexa-hydro-S-triazine, Hexamethylentetramin, N-Hydroxymethyl-N'-methylthioharnstoff, Methylenbismorpholin, Natrium N-(Hydroxymethyl)glycinat, N-Methylolchloracetamid, Oxazolidine, Paraformaldehyd, Taurolin, Tetrahydro-1,3-oxazin, Tetra-methylol-acetylen-diharnstoff (TMAD);
Isothiazolinone wie:
   N-Methylisothiazolin-3-on, 5-Chlor-N-methylisothiazolin-3-on, 4,5-Dichloro-N-octylisothiazolin-3-on, 5-Chlor-N-octylisothiazolinon, N-Octyl-isothiazolin-3-on, 4,5-Trimethylenisothiazolinon, 4,5-Benzisothiazolinon;
Aldehyde wie:
   Zimtaldehyd, Formaldehyd, Glutardialdehyd, β-Bromzimtaldehyd, o-Phthaldialdehyd;
Thiocyanate wie:
   Thiocyanatomethylthiobenzothiazol, Methylenbisthiocyanat;
quartäre Ammoniumverbindungen und Guanidine wie:
   Benzalkoniumchlorid, Benzyldimethyltetradecylammoniumchlorid, Benzyldimethyldodecylammoniumchlorid, Dichlorbenzyldimethylalkylammoniumchlorid, Didecyldimethylammoniumchlorid, Dioctyldimethylammoniumchlorid, N-Hexadecyltrimethylammoniumchlorid, 1 Hexadecylpyridiniumchlorid, Iminoctadine-tris(albesilat);
Phenole wie:
   Tribromphenol, Tetrachlorphenol, 3-Methyl-4-chlorphenol, 3,5-Dimethyl-4-chlorphenol, Dichlorphen, 2-Benzyl-4-chlorphenol, Triclosan, Diclosan, Hexachlorophen, p-Hydroxybenzoesäuremethylester, p-Hydroxybenzoesäureethylester, p-Hydroxybenzoesäurepropylester, p-Hydroxybenzoesäurebutylester, p-Hydroxybenzoesäureoctylester ,o-Phenylphenol, m-Phenylphenol, p-Phenylphenol, 4-(2-tert.-Butyl-4-methyl-phenoxy)-phenol, 4-(2-Isopropyl-4-methyl-phenoxy)-phenol, 4-(2,4-Dimethyl-phenoxy)-phenol und deren Alkali- und Erdalkalimetallsalze;
Mikrobizide mit aktivierter Halogengruppe wie:
   Bronopol, Bronidox, 2-Brom-2-nitro-1,3-propandiol, 2-Brom-4'-hydroxy-acetophenon, 1-Brom-3-chlor-4,4,5,5-tetramethyl-2-imidazoldinone, β-Brom-β-nitrostyrol, Chloracetamid, Chloramin T, 1,3-Dibrom-4,4,5,5-tetrametyl-2-imidazoldinone, Dichloramin T, 3,4-Dichlor-(3H)-1,2-dithiol-3-on, 2,2-Dibrom-3-nitril-propionamid, 1,2-Dibrom-2,4-dicyanobutan, Halane, Halazone, Mucochlorsäure, Phenyl-(2-chlor-cyanvinyl)sulfon, Phenyl-(1,2-dichlor-2-cyanvinyl)sulfon, Trichlorisocyanursäure;
Pyridine wie:
   1-Hydroxy-2-pyridinthion (und ihre Cu-, Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;
Methoxyacrylate oder Ähnliche wie:
   Azoxystrobin, Dimoxystrobin, Fluoxastrobin, Kresoxim-methyl, Metominostrobin, Orysastrobin, Picoxystrobin, Pyraclostrobin, Trifloxystrobin, 2,4-dihydro-5-methoxy-2-methyl-4-[2-[[[[1-[3-(trifluoromethyl)phenyl]ethylidene]amino]oxy]methyl]phenyl]-3H-1,2,4-triazol-3-one (CAS-Nr. 185336-79-2);
Metallseifen wie:
   Salze der Metalle Zinn, Kupfer und Zink mit höheren Fett-, Harz-, Naphthensäuren und Phosphorsäure wie z.B. Zinn-, Kupfer-, Zinknaphtenat, -octoat, 2-ethylhexanoat, -oleat, phosphat, -benzoat;
Metallsalze wie:
   Salze der Metalle Zinn, Kupfer, Zink, sowie auch Chromate und Dichromate wie z.B. Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat;
Oxide wie:
   Oxide der Metalle Zinn, Kupfer und Zink wie z.B. Tributylzinnoxid, Cu2O, CuO, ZnO;
Oxidationsmittel wie:
   Wasserstoffperoxid, Peressigsäure, Kalium-persulfat;
Dithiocarbamate wie:
   Cufraneb, Ferban, Kalium-N-hydroxymethyl-N'-methyl-dithiobarbamat, Na- oder K-dimethyldithiocarbamat, Macozeb, Maneb, Metam, Metiram, Thiram, Zineb, Ziram;
Nitrile wie:
   2,4,5,6-Tetrachlorisophthalodinitril, Dinatrium-cyano-dithioimidocarbamat;
Chinoline wie:
   8-Hydroxychinolin und deren Cu-Salze;
sonstige Fungizide und Bakterizide wie:
   Bethoxazin, 5-Hydroxy-2(5H)-furanon; 4,5-Benzdithiazolinon, 4,5-Trimethylendithiazolinon, N (2-p-Chlorbenzoylethyl)-hexaminiumchlorid, 2-Oxo-2-(4-hydroxy-phenyl)-acethydroximsäure-chlorid, Tris-N-(cyclohexyldiazeniumdioxy)-aluminium, N-(Cyclo-hexyldiazeniumdioxy)-tributylzinn bzw. K-Salze, Iprovalicarb, Fenhexamid, Spiroxamin, Carpropamid, Diflumetorin, Quinoxyfen, Famoxadone, Polyoxorim, Acibenzolar-S-methyl, Furametpyr, Thifluzamide, Methalaxyl-M, Benthiavalicarb, Metrafenon, Cyflufenamid, Tiadinil, Teebaumöl, Phenoxyethanol,
Ag, Zn oder Cu-haltige Zeolithe allein oder eingeschlossen in polymere Werkstoffe.

Ganz besonders bevorzugt sind Mischungen mit
Azaconazol, Bromuconazol, Cyproconazol, Dichlobutrazol, Diniconazol, Diuron, Hexacona-zol, Metaconazol, Penconazol, Propiconazol, Tebuconazol, Dichlofluanid, Tolylfluanid, Fluorfolpet, Methfuroxam, Carboxin, Benzo[b]thiophen-S,S-dioxid-carbon-säurecyclohexylamid, Fenpiclonil, 4-(2,2-Difluoro-1,3-benzodioxol-4-yl)-1H-pyrrol-3-carbo-nitril, Butenafin, Imazalil, N-Methyl-isothiazolin-3-on, 5-Chlor-N-methylisothiazolin-3-on, N-Octylisothiazolin-3-on, Dichlor-N-octylisozhiazolinon, Mercaptobenzthiazol, Thiocyanatomethylthiobenzothiazol, Thiabendazol, Benzisothiazolinon, Benzylalkohol-(hemi)-formal, N Methylolchloracetamid, Glutaraldehyd, Omadine, Zn Omadine, Dimethyldicarbonat, 2-Brom-2-nitro-1,3-propandiol, Bethoxazin, o-Phthaldialdehyd, 2,2-Dibrom-3-nitril-propionamid, 1,2-Dibrom-2,4-dicyanobutan, 1,3-Bis(hydroxymethyl)-5,5-dimethylimidazolidine-2,4-dione (DMDMH), Tetra¬methylol-acetylen-diharnstoff (TMAD), Ethylenglycol-hemiformal, p-Hydroxybenzoesäure, Carbendazim, Chlorophen, 3-Methyl-4-chlorphenol, o-Phenylphenol.

Desweiteren werden neben den oben genannten Fungiziden und Bakteriziden auch gut wirksame Mischungen mit anderen Wirkstoffen hergestellt:
Insektizide / Akarizide / Nematizide:
   Abamectin, Acephat, Acetamiprid, Acetoprole, Acrinathrin, Alanycarb, Aldicarb, Aldoxycarb, Aldrin, Allethrin, Alpha-cypermethrin, Amidoflumet, Amitraz, Avermectin, Azadirachtin, Azinphos A, Azinphos M, Azocyclotin,
   Bacillus thuringiensis, Barthrin, 4-Bromo-2(4-chlorphenyl)-1-(ethoxymethyl)-5-(trifluoro-methyl)-1H-pyrrole-3-carbonitrile, Bendiocarb, Benfuracarb, Bensultap, Betacyfluthrin, Bifenthrin, Bioresmethrin, Bioallethrin, Bistrifluron, Bromophos A, Bromophos M, Bufencarb, Buprofezin, Butathiophos, Butocarboxin, Butoxycarboxim,
   Cadusafos, Carbaryl, Carbofuran, Carbophenothion, Carbosulfan, Cartap, Chinomethionat, Cloethocarb, Chlordane, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamid, Chlorpicrin, Chlorpyrifos A, Chlorpyrifos M, Cis-Resmethrin, Clocythrin, Clothiazoben, Cypophenothrin Clofentezin, Coumaphos, Cyanophos, Cycloprothrin, Cyfluthrin, Cyhalothrin, Cyhexatin, Cypermethrin, Cyromazin,
   Decamethrin, Deltamethrin, Demeton M, Demeton S, Demeton-S-methyl, Diafenthiuron, Dialiphos, Diazinon, 1,2-Dibenzoyl-1(1,1-dimethyl)-hydrazin, DNOC, Dichlofenthion, Dichlorvos, Dicliphos, Dicrotophos, Difethialon, Diflubenzuron, Dimethoat, 3,5-Dimethylphenyl-methylcarbamat, Dimethyl-(phenyl)-silyl-methyl-3-phenoxybenzylether, Dimethyl-(4-Ethoxyphenyl)-silylmethyl-3-phenoxybenzylether, Dimethylvinphos, Dioxathion, Disulfoton,
   Eflusilanate, Emamectin, Empenthrin, Endosulfan, EPN, Esfenvalerat, Ethiofencarb, Ethion, Ethofenprox, Etrimphos, Etoxazole, Etobenzanid,
   Fenamiphos, Fenazaquin, Fenbutatinoxid, Fenfluthrin, Fenitrothion, Fenobucarb, Fenothiocarb, Fenoxycarb, Fenpropathrin, Fenpyrad, Fenpyroximat, Fensulfothion, Fenthion, Fenvalerate, Fipronil, Flonicamid, Fluacrypyrim, Fluazuron, Flucycloxuron, Flucythrinate, Flufenerim, Flufenoxuron, Flupyrazofos, Flufenzine, Flumethrin Flufenprox, Fluvalinate, Fonophos, Formethanate, Formothion, Fosmethilan Fosthiazat, Fubfenprox, Furathiocarb,
   Halofenocid, HCH (CAS RN: 58-89-9), Heptenophos, Hexaflumuron, Hexythiazox, Hydramethylnon, Hydropren,
   Imidacloprid, Imiprothrin, Indoxycarb, Iprinomectin, Iprobenfos, Isazophos, Isoamidophos, Isofenphos, Isoprocarb, Isoprothiolane, Isoxathion, Ivermectin,
   Kadedrin
   Lambda-Cyhalothrin, Lufenuron,
   Malathion, Mecarbam, Mervinphos, Mesulfenphos, Metaldehyd, Methacrifos, Methamidophos, Methidathion, Methiocarb, Methomyl, Metalcarb, Milbemectin, Monocrotophos, Moxiectin,
   Naled, NI 125, Nicotin, Nitenpyram, Noviflumuron,
   Omethoat, Oxamyl, Oxydemethon M, Oxydeprofos,
   Parathion A, Parathion M, Penfluron, Permethrin, 2-(4-Phenoxyphenoxy)-ethyl-ethyl-carbamat, Phenthoat, Phorat, Phosalon, Phosmet, Phosphamidon, Phoxim, Pirimicarb, Pirimiphos M, Pirimiphos A, Prallethrin, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyridalyl, Pyrimidifen, Pyriproxifen, Pyrithiobac-natrium Quinalphos,
   Resmethrin, Rotenon,
   Salithion, Sebufos, Silafluofen, Spinosad, Spirodiclofen, Spiromesifen, Sulfotep, Sulprofos,
   Tau-fluvalinat, Taroils, Tebufenozide, Tebufenpyrad, Tebupirimphos, Teflubenzuron, Tefluthrin, Temephos, Terbam, Terbufos, Tetrachlorvinphos, Tetramethrin, Tetramethacarb, Thiacloprid, Thiafenox, Thiamethoxam, Thiapronil, Thiodicarb, Thiofanox, Thiazophos, Thiocyclam, Thiomethon, Thionazin, Thuringiensin, Tralomethrin, Transfluthrin, Triarathen, Triazophos, Triazamate, Triazuron, Trichlorfon, Triflumuron, Trimethacarb,
   Vamidothion, Xylylcarb, Zetamethrin;
Molluscizide:
   Fentinacetat, Metaldehyd, Methiocarb, Niclosamid;
Herbizide und Algizide:
   Acetochlor, Acifluorfen, Aclonifen, Acrolein, Alachlor, Alloxydim, Ametryn, Amidosulfuron, Amitrole, Ammonium sulfamate, Anilofos, Asulam, Atrazine, Azafenidin, Aziptrotryn, Azimsulfuron,
   Benazolin, Benfluralin, Benfuresat, Bensulfuron, Bensulfid, Bentazon, Benzofencap, Benzthiazuron, Bifenox, Bispyribac, Bispyribac-Natrium, Borax, Bromacil, Bromobutide, Bromofenoxim, Bromoxynil, Butachlor, Butamifos, Butralin, Butylat, Bialaphos, Benzoylprop, Bromobutide, Butroxydim,
   Carbetamid, Carfentrazone-ethyl, Carfenstrol, Chlomethoxyfen, Chloramben, Chlorbromuron, Chlorflurenol, Chloridazon, Chlorimuron, Chlornitrofen, Chloressigsäure, Chloransulam-methyl, Cinidon-ethyl, Chlorotoluron, Chloroxuron, Chlorpropham, Chlorsulfuron, Chlorthal, Chlorthiamid, Cinmethylin, Cinofulsuron, Clefoxydim, Clethodim, Clomazone, Chlomeprop, Clopyralid, Cyanamide, Cyanazine, Cycloat, Cycloxydim, Chloroxynil, Clodinafop-propargyl, Cumyluron, Clometoxyfen, Cyhalofop, Cyhalofop-butyl, Clopyrasuluron, Cyclosulfamuron,
   Diclosulam, Dichlorprop, Dichlorprop-P, Diclofop, Diethatyl, Difenoxuron, Difenzoquat, Diflufenican, Diflufenzopyr, Dimefuron, Dimepiperate, Dimethachlor, Dimethipin, Dinitramine, Dinoseb, Dinoseb Acetate, Dinoterb, Diphenamid, Dipropetryn, Diquat, Dithiopyr, Diduron, DNOC, DSMA, 2,4-D, Daimuron, Dalapon, Dazomet, 2,4-DB, Desmedipham, Desmetryn, Dicamba, Dichlobenil, Dimethamid, Dithiopyr, Dimethametryn,
   Eglinazin, Endothal, EPTC, Esprocarb, Ethalfluralin, Ethidimuron, Ethofumesat, Ethobenzanid, Ethoxyfen, Ethametsulfuron, Ethoxysulfuron,
   Fenoxaprop, Fenoxaprop-P, Fenuron, Flamprop, Flamprop-M, Flazasulfuron, Fluazifop, Fluazifop-P, Fuenachlor, Fluchloralin, Flufenacet Flumeturon, Fluorocglycofen, Fluoronitrofen, Flupropanate, Flurenol, Fluridone, Flurochloridone, Fluroxypyr, Fomesafen, Fosamine, Fosametine, Flamprop-isopropyl, Flamprop-isopropyl-L, Flufenpyr, Flumiclorac-pentyl, Flumipropyn, Flumioxzim, Flurtamon, Flumioxzim, Flupyrsulfuronmethyl, Fluthiacet-methyl,
   Glyphosate, Glufosinate-ammonium
   Haloxyfop, Hexazinon,
   Imazamethabenz, Isoproturon, Isoxaben, Isoxapyrifop, Imazapyr, Imazaquin, Imazethapyr, loxynil, Isopropalin, Imazosulfuron, Imazomox, Isoxaflutole, Imazapic,
   Ketospiradox,
   Lactofen, Lenacil, Linuron,
   MCPA, MCPA-hydrazid, MCPA-thioethyl, MCPB, Mecoprop, Mecoprop-P, Mefenacet, Mefluidid, Mesosulfuron, Metam, Metamifop, Metamitron, Metazachlor, Methabenzthiazuron, Methazol, Methoroptryne, Methyldymron, Methylisothiocyanat, Metobromuron, Metoxuron, Metribuzin, Metsulfuron, Molinat, Monalid, Monolinuron, MSMA, Metolachlor, Metosulam, Metobenzuron,
   Naproanilid, Napropamid, Naptalam, Neburon, Nicosulfuron, Norflurazon, Natriumchlorat,
   Oxadiazon, Oxyfluorfen, Oxysulfuron, Orbencarb, Oryzalin, Oxadiargyl,
   Propyzamid, Prosulfocarb, Pyrazolate, Pyrazolsulfuron, Pyrazoxyfen, Pyribenz¬oxim, Pyributicarb, Pyridat, Paraquat, Pebulat, Pendimethalin, Pentachlorophenol, Pentoxazon, Pentanochlor, Petroleumöle, Phenmedipham, Picloram, Piperophos, Pretilachlor, Primisulfuron, Prodiamine, Profoxydim, Prometryn, Propachlor, Propanil, Propaquizafob, Propazine, Propham, Propisochlor, Pyriminobac-methyl, Pelargonsäure, Pyrithiobac, Pyraflufen-ethyl,
   Quinmerac, Quinocloamine, Quizalofop, Quizalofop-P, Quinchlorac,
   Rimsulfuron
   Sethoxydim, Sifuron, Simazine, Simetryn, Sulfosulfuron, Sulfometuron, Sulfentrazone, Sulcotrione, Sulfosate,
   Teeröle, TCA, TCA-Natrium, Tebutam, Tebuthiuron, Terbacil, Terbumeton, Terbutylazine, Terbutryn, Thiazafluoron, Thifensulfuron, Thiobencarb, Thiocarbazil, Tralkoxydim, Triallate, Triasulfuron, Tribenuron, Triclopyr, Tridiphane, Trietazine, Trifluralin, Tycor, Thdiazimin, Thiazopyr, Triflusulfuron, Vernolat.

Von der Erfindung ist ebenfalls ein Verfahren zur Herstellung von penflufenhaltigen Polymerpartikeln umfasst, bei dem, als ein ethylenisch ungesättigter Monomeren ein neutraler, ungeladener, ethylenisch ungesättigter Monomeren des Typs M1 ausgewählt aus der Gruppe Styrol, Divinylbenzol, Ester monoethylenisch ungesättigter Mono- und Dicarbonsäuren mit 3 bis 8 und insbesondere 3 oder 4 C-Atomen mit C₁-C₂₀-Alkanolen oder mit C₅-C₈-Cycloalkanolen in einer Menge von mindestens 60 Gew. %, bezogen auf die Gesamtmenge der eingesetzten Monomeren, in Gegenwart von Penflufen oder dessen Salze und Säureadditionsverbindungen und in Gegenwart eines öllöslichen radikalischen Initiators in einer Öl-in-Wasser Emulsionspolymerisation polymerisiert wird.

Die Herstellung der erfindungsgemäßen penflufenhaltigen Polymerpartikel erfolgt üblicherweise so, dass zunächst eine Emulsion hergestellt wird. Im Allgemeinen werden zur Herstellung dieser Emulsion zunächst die Monomeren und Penflufen oder dessen Salze und Säureadditionsverbindungen, gegebenenfalls in Gegenwart weiterer Hilfsstoffe, vermischt. Die Zugabe kann dabei so erfolgen, dass in einem Schritt, die vollständige Menge der Monomeren und des Penflufens zu der wässrigen Lösung gegeben wird. Sie kann aber auch dosiert erfolgen. Im Falle der dosierten Zugabe erfolgt die Zugabe in der Regel mindestens über einen Zeitraum von 0,5 h. Bevorzugt erfolgt die Zugabe in einem Zeitraum von 0,5 bis 5 Stunden. Gegebenenfalls wird ein neutraler pH-Wert eingestellt. Die Öl-in-Wasser Emulsion aus Monomeren, Penflufen und gegebenenfalls niedermolekularen Emulgatoren, Schutzkolloiden und Costabilisatoren wird in der Regel unter Erzeugung von hohen Scherkräften in eine Emulsion mit Partikeln der gewünschten Größe umgewandelt. Um hohe Scherkräfte zu erzeugen, werden im Allgemeinen Rotor Stator Systeme wie Ultraturrax, Ultraschall, Hochdruckdispergatoren oder axial durchströmte Düsenaggregate verwendet. Üblicherweise wird dann der radikalische Initiator zugegeben. Der radkalische Iniator kann aber genauso gut der Mischung aus Monomeren und Penflufen zugesetzt worden sein, bevor die Mischung emulgiert wird. In der Regel wird die entstandenen Emulsion dann erwärmt. Üblicherweise wird die erwärmte Emulsion für einen Zeitraum von 1 bis 20 Stunden, bevorzugt 8 bis 15 Stunden, nachgerührt. Die entstandene Suspension der erfindungsgemäßen penflufenhaltigen Polymerpartikel bedarf in der Regel keiner weiteren Aufarbeitung. Die wässrige Suspension der erfindungsgemäßen Polymerpartikel kann daher in der vorliegenden Form zum Schutz von technischen Materialien eingesetzt werden. Die penflufenhaltigen Polymerpartikel können aber auch isoliert und getrocknet werden und dann durch Zugabe von Lösungs- und Verdünnugsmitteln zu penflufenhaltigen Mitteln verabeitet werden oder die wässrige Suspension der erfindungsgemäßen penflufenhaltigen Polymerpartikel kann durch Zugabe von Verdickungsmitteln, Entschäumern oder Gebindekonservierungsmittel zu penflufenhaltigen Mitteln umgesetzt werden. Die Isolierung der penflufenhaltigen Polymerpartikel kann z.B. durch Filtration und Verdampfen des Wassers, beispielsweise im Sprühtrockner, erfolgen. Falls innerhalb des erfindungsgemäßen Herstellungsverfahren der penflufenhaltigen Polymerpartikel der pH-Wert eingestellt werden muss, dann erfolgt die Einstellung des pH-Wertes üblicherweise unter Verwendung von Alkalimetallsalze von Carbonaten Hydrogencarbonaten, Phosphaten, Hydrogenphosphaten, Dihydrogenphoshaten, Citraten oder durch Verwendung Alkalimetallverbindungen schwacher organischer Säuren.

Bevorzugt erfolgt die erfindungsgemäße Herstellung der penflufenhaltigen Polymerpartikel so, dass zunächst die wässrige Lösung, bevorzugt niedermolekulare Emulgatoren enthaltend, vorgelegt wird. Eine weitere Mischung, die die Monomeren und die Wirkstoffe, insbesondere Penflufen enthält, und gegebenenfalls weiterer Hilfsstoffe, und radikalische Initiatoren wird dann entweder dosiert oder auch im Wesentlichen in einer Menge, zu der wässrigen Lösung gegeben. Dann wird bevorzugt der radikalische Initiator zugegeben. Bevorzugt wird danach die Emulsion unter hohen Scherkräften emulgiert, um auf die gewünschte Partikelgröße zu kommen. Die Emulsion wird vorzugsweise dann erwärmt und für einen Zeitraum von vorzugsweise 8 bis 15 Stunden nachgerührt. Bevorzugt wird die wässrige Suspension der penflufenhaltigen Polymerpartikel dann unter Verwendung von Verdickungsmitteln, Entschäumern oder Gebindekonservierungsmittel zu penflufenhaltigen Mitteln verarbeitet.

Die Polymerisation erfolgt im Allgemeinen bei Temparaturen zwischen 50 °C und 90 °C. Die Polymerisation kann aber auch bei höheren oder geringeren Temperaturen durchgeführt werden. Bevorzugt erfolgt die Polymerisation bei 60 °C bis 80 °C.

Im Allgemeinen werden 0,1 Gew. % bis 30 Gew.% , bevorzugt 0,2 Gew.% bis 20 Gew.% und ganz besonders bevorzugt 0,5 Gew.% bis 15 Gew.% Penflufen bezogen auf die Gesamtmenge der Monomeren im erfindungsgemäßen Herstellungsverfahren der penflufenhaltigen Polymerpartikel eingesetzt.

Die Molekulargewichte der erfindungsgemäßen penflufenhaltigen Polymerpartikel können in einem sehr breiten Bereich variieren, bevorzugt haben sie eine Molmasse von 10.000 bis 100.000 g/mol.

Die erfindungsgemäßen penflufenhaltigen Polymerpartikel weisen üblicherweise einen mittleren Teilchendurchmesser von kleiner 1,5 µm auf, bevorzugt von 100 nm bis 1 µm, besonders bevorzugt 350 nm bs 600 nm auf. Die Bestimmung des mittleren Teilchendurchmessers erfolgt durch Laserbeugung gemäß ISO 13320-1.

Wenn innerhalb der Patentanmeldung auf eine Ölphase Bezug genommen wird, dann ist hierbei die Phase gemeint, in der sich die im Wesentlichen wasserunlöslichen, eingesetzten Verbindungen gegenseitig gelöst haben. Die Ölphase enthält im Allgemeinen das oder die Monomeren, Penflufen, radikalischer öllöslicher Initiator, ggf. einen Costabilisator, ggf. weitere Wirkstoffe und ggf. weitere Hilfsstoffe.

Die Wasserphase enthält im Allgemeinen einen oder mehrere Emulgatoren und/oder Schutzkolloide und ggf. Salze und Radikalfänger, die verhindern sollen, dass die Polymerisation in der Wasserphase startet.

Das Verhältnis von Ölphase zu Wasserphase kann in einem großen Bereich variiert werden. Im Allgemeine beträgt das Verhältnis 10:1 bis 1:10, bevorzugt 5:1 bis 1:5.

Die erfindungsgemäßen penflufenhaltigen Polymerpartikel können durch Zugabe von weiteren Hilfsmitteln, wie z.B. Lösungs- und Verdünnungsmitteln, Antioxidantien, Radikalfänger, UV-Stabilisatoren, wie UV-Absorbern und Chelatoren, Entschäumern, sowie weitere Bioziden, Verdickungsmitteln und Gebindekonservierungsmitteln zu penflufenhaltigen Mitteln umgesetzt werden oder auch direkt ohne weitere Zugabe dieser Inhaltsstoffe eingesetzt werden. Bevorzugt werden den erfindungsgemäßen penflufenhaltigen Polymerpartikeln, Lösungs- und Verdünnungsmitteln und gegebenenfalls dann Verdickungsmittel, Entschäumer und Gebindekonservierungsmittel zugesetzt.

Die penflufenhaltigen Mittel können in bekannter Weise hergestellt werden, z.B. durch Vermischen der penflufenhaltigen Polymerpartikel mit Lösungs- und Verdünnungsmitteln oder unter Druck stehenden verflüssigten Gasen, gegebenenfalls unter Verwendung von Verdickungsmitteln, Entschäumern und Gebindekonservierungsmitteln. Bevorzugt werden die penflufenhaltigen Mittel durch Zugabe von Verdickungsmitteln, Entschäumern und Gebindekonservierungsmittel zu der wässrigen Suspension der penflufenhaltigen Polymerisaten gegeben. Als Lösungs- und Verdünnungsmitteln kommen im Wesentlichen in Frage: Aromaten, wie Xylol, Toluol oder Alkylnaphthaline, chlorierte Aromaten oder chlorierte aliphatische Kohlenwasserstoffe, wie Chlorbenzole oder Chlorethylene, aliphatische Kohlenwasserstoffe, wie Cyclohexan oder Paraffine, z.B. Erdölfraktionen, Alkohole, wie Butanol oder Glycol sowie deren Ether und Ester, sowie Wasser.

Als Verdickungsmittel werden beispielweise Polysaccharide, Xanthan Gum, Natrium- oder Magnesium Silicate, Heteropolysaccharide, Alginate, Carboxymethylcellulose, Gummi Arabicum oder Polyacrylsäuren eingesetzt. Bevorzugt wird als Verdickungsmittel Xanthan Gum eingesetzt.

Als Gebindekonservierungsmittel werden beispielsweise und vorzugsweise Biozide, Bakterizide und Fungizide eingesetzt.

Als Entschäumer können im Allgemeinen grenzflächenaktive Stoffe eingesetzt werden, die in der tensidischen Lösung nur schwach löslich sind. Bevorzugte Entschäumer sind solche, die sich von natürlichen Fetten und Ölen, Petroleum-Derivaten oder Siliconölen ableiten.

Die penflufenhaltige Mittel könnten auch eingefärbt werden. Als Farbstoffe können anorganische Pigmente, wie z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die penflufenhaltigen Mittel enthalten im Allgemeinen zwischen 0.1 und 90 Gewichtsprozent penflufenhaltige Polymerpartikel, vorzugsweise zwischen 2 und 75 Gewichtsprozent.

Gegenstand der vorliegenden Erfindung sind daher penflufenhaltige Mittel auf Basis der erfindungsgemäßen penflufenhaltigen Polymerpartikel, enthaltend mindestens ein Lösungs- oder Verdünnungsmittel, sowie gegebenenfalls Verdickungsmittel, gegebenenfalls Entschäumer und gegebenenfalls Gebindekonservierungsmittel und gegebenenfalls weitere antimikrobiell wirksame Stoffe. Irn Materialschutz lassen sich die erfindungsgemäßen penflufenhaltigen Polymerpartikel und die penflufenhaltigen Mittel zum Schutz von technischen Materialien gegen Befall und Zerstörung durch unerwünschte Mikroorganismen einsetzen. Unter technischen Materialien sind im vorliegenden Zusammenhang nichtlebende Materialien zu verstehen, die für die Verwendung in der Technik zubereitet worden sind. Beispielsweise können technische Materialien, die durch Penflufen vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt sind Holz, Holzprodukte und Holz-Plastik-Komposite.

Von der Erfindung ist daher auch die Verwendung der erfindungsgemäßen penflufenhaltigen Polymerpartikel und der penflufenhaltigen Mittel zum Schutz von technischen Materialien umfasst.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirkt Penflufen gegen Pilze, insbesondere Schimmelpilze, holzverfärbende und holzzerstörende Pilze (Basidiomyceten) sowie gegen Schleimorganismen urid Algen. Des Weiteren wurde gefunden, dass die erfindungsgemäßen penflufenhaltigen Polymerpartikel und penflufenhaltigen Mittel, einen hervorragenden Schutz des Holzes gegen holzzerstörendee Pilze (Basidiomyceten) aufweisen.

Von der Erfindung ist daher auch die Verwendung der erfindungsgemäßen penflufenhaltigen Polymerpartikel und der penflufenhaltigen Mittel zum Schutz von Holz, Holzprodukten und Holz-Plastik-Komposite gegen die Zerstörung durch Mikroorganismen umfasst.

Es seien beispielsweise Mikroorganismen der folgenden Gattungen genannt:
Altemaria, wie Altemaria tenuis,
Aspergillus, wie Aspergillus niger,
Chaetomium, wie Chaetomium globosum,
Coniophora, wie Coniophora puetana,
Fomitopsis, wie Fomitopsis palustris
Gloeophyllum, wie Gloeophyllum trabeum,
Lentinus, wie Lentinus tigrinus,
Poria, wie Poria placenta,
Penicillium, wie Penicillium glaucum,
Polyporus, wie Polypom versicolor,
Aureobasidium, wie Aureobasidium pullulans,
Sclerophoma, wie Sclerophoma pityophila,
Stereum, wie Stereum sanguinolentum.
Trichoderma, wie Trichoderma viride,
Escherichia, wie Escherichia coli,
Pseudomonas, wie Pseudomonas aeruginosa,
Staphylococcus, wie Staphylococcus aureus.

Als holzzerstörende Basidiomyceten, die einen Abbau oder eine Veränderung von Holz und holzhaltigen Materialien bewirken können, seien beispielsweise und vorzugsweise genannt:
Coniophora, wie Coniophora puteana,
Lentinus, wie Lentinus tigrinus, Antrodia, wie Antrodia sinuosa Polyporus, wie Polyporus versicolor,
Gloeophyllum, wie Gloeophyllum trabeum,
Fomitopsis, wie Fomitopsis palustris
Poria, wie Poria placenta,
Stereum, wie Stereum sanguinolentum.

Besonders bevorzugt sind die holzzerstörenden Basidiomyceten, insbesondere Holobasidiomyceten. Holzzerstörende Basidiomyceten und Holobasidiomyceten sind Pilze.

Die erfindungsgemäßen penflufenhaltigen Polymerpartikel zeigen eine hervorragende Wirkung gegenüber Pilzen, insbesondere gegenüber holzzerstörenden Pilzen.

Von der Erfindung ist daher auch die Verwendung der erfindungsgemäßen penflufenhaltigen Polymerpartikel und der penflufenhaltigen Mittel zum Schutz von Holz, Holzprodukten und Holz-Plastik-Komposite gegen die Zerstörung durch holzzerstörenden Basidiomyceten umfasst.

Ganz besonders bevorzugt wirken die penflufenhaltigen Polymerpartikel und die penflufenhaltigen Mittel gegen Arten der Gattungen Gloeophyllum, Coniophora, Coriolus, Stereum oder Poria. Noch weiter bevorzugt wirken die penflufenhaltigen Polymerpartikel gegen Arten der Gattungen Coniphora oder Poria, insbesondere gegen Poria placenta und Coniphora puteana. Noch weiter bevorzugt ist die Verwendung von penflufenhaltigen Polymerpartikeln zum Schutz von Holz gegen Poria placenta.

Die erfindungsgemäßen penflufenhaltigen Polymerpartikel und penflufenhaltigen Mittel lassen sich mit Hilfe der üblicherweise verwendeten Applikationsmethoden, wie Vakuum-, Doppelvakuum-, Vakuumdruck- oder Druckverfahren sehr gut in technische Materialien, insbesondere in Holz und Holzprodukte einarbeiten.

Unter Holz wird insbesondere verstanden: Bauholz, Holzbalken, Eisenbahnschwellen, Brückenteile, Bootsstege, Holzfahrzeuge, Kisten, Paletten, Container, Telefonmasten, Holzzäune, Holzverkleidungen, Holzfenster und -türen, Tischlerarbeiten und Holzprodukte, die beim Hausbau oder in der Bautischlerei Verwendung finden.

Im Rahmen des erfindungsgemäßen Herstellungsverfahren lassen sich erstmals stabile penflufenhaltige Polymerpartikel herstellen. Zudem weisen die erfindungsgemäßen penflufenhaltigen Polymerpartikel und penflufenhaltigen Mittel eine gute Langzeitwirkung zum Schutz von technischen Materialien, insbesondere von Holz, Holzprodukten und Holz-Plastik-Komposite auf.

### Beispiele:

Alle angegebenen Teilchengrößen wurde mit einen Teilchenmessgerät Beckmann Coulter LS 13320 mit PIDS Modul gemäß ISO 13320-1 durchgeführt.

### Beispiel 1

Eine Lösung von 2,88 g SDS (Na-Dodecylsulfat), 0,163 g Natriumhydrogencarbonat in 300g Wasser wird mit einer organischen Lösung bestehend aus 91,7 g Methylmethacrylat, 4,3 g Penflufen, 3,84 g Hexadecan, 1,92 g Benzylalkohol und 1,63 g 2,2'-Azobis(2-methylbutyronitril) versetzt und zum Erzeugen einer Emulsion 30 min mit einem Ultraturrax (IKA T 25 digital, 11000 U/min) behandelt. Die so erhaltene Emulsion wird in einen 500 ml Edelstahlbecher überführt und unter Rühren und Kühlung (Eisbad) 30 min mit einem Ultraschallfinger mit Ultraschall beschallt (Hielscher Ultrasonics; Ultraschallprozessor UP200St mit Sonotrode S26d7, Amplitude 90 %). Anschließend überführt man in einen 1000 ml Dreihalskolben, wäscht mit N₂ und erhitzt unter Rühren langsam auf 70 °C. Es wird bei dieser Temperatur 15 h nachgerührt. Nach Abkühlen erhält man eine dünnflüssige, weißlich blaue Suspension mit einem Gehalt an Penflufen von 1,02 % (HPLC).

Mittlere Teilchengröße (gemessen mit Laserbeugung) 0,091 µm ; 90 % der Teilchen (Vol%) sind kleiner 0,128 µm.

### Beispiel 2

Eine Lösung von 2,88 g SDS (Na-Dodecylsulfat), 0,163 g Natriumhydrogencarbonat in 200g Wasser wird mit einer organischen Lösung bestehend aus 91,7 g Methylmethacrylat, 1,92 g Divinylbenzol, 4,3 g Penflufen, 3,84 g Hexadecan und 1,63 g 2,2'-Azobis(2-methylbutyronitril) versetzt und zum Erzeugen einer Emulsion 15 min durch einen Ultraturrax (IKA T 25 digital, 10800 U/min) laufen gelassen (im Kreis). Anschließend überführt man in einen 1000 ml Dreihalskolben, wäscht mit N₂ und erhitzt unter Rühren langsam auf 70 °C. Es wird bei dieser Temperatur 15 h nachgerührt. Nach dem Abkühlen erhält man eine dünnflüssige, weiße Suspension, enthaltend 1,81 % Penflufen (HPLC).

Mittlere Teilchengröße (gemessen mit Laserbeugung) 0,571 µm; 90 % der Teilchen (Vol%) sind kleiner 1,193 µm.

### Beispiel 3

Eine Lösung von 2,88 g SDS (Na-Dodecylsulfat), 0,163 g Natriumhydrogencarbonat in 200g Wasser wird mit einer organischen Lösung bestehend aus 91,7 g Methylmethacrylat, 1,92 g Divinylbenzol, 4,3 g Penflufen, 3,84 g Hexadecan und 1,63 g 2,2'-Azobis(2-methylbutyronitril) versetzt und zum Erzeugen einer Emulsion 15 min durch einen Durchlaufultraturrax (IKA T 25 digital / Aufsatz DK 25.11; 10800 U/min) laufen gelassen (im Kreis). Anschließend überführt man in einen 1000 ml Dreihalskolben, wäscht mit N₂ und erhitzt unter Rühren langsam auf 70 °C. Es wird bei dieser Temperatur 15 h nachgerührt. Nach dem Abkühlen erhält man eine dünnflüssige, weißliche Suspension mit einem Gehalt an Penflufen von 1,51 % (HPLC).

Mittlere Teilchengröße (gemessen mit Laserbeugung) 0,571 µm ; 90 % der Teilchen (Vol%) sind kleiner 1,193 µm.

### Beispiel 4

Eine Lösung von 2,88 g SDS (Na-Dodecylsulfat), 0,163 g Natriumhydrogencarbonat in 200g Wasser wird mit einer organischen Lösung bestehend aus 45,85 g Methylmethacrylat, 45,85 g n-Buthylmethacrylat, 4,3 g Penflufen, 3,84 g Hexadecan und 1,63 g 2,2'-Azobis(2-methylbutyronitril) versetzt und zum Erzeugen einer Emulsion 15 min durch einen Durchlaufultraturrax (IKA T 25 digital / Aufsatz DK 25.11; 10800 U/min) laufen gelassen (im Kreis). Anschließend überführt man in einen 1000 ml Dreihalskolben, wäscht mit N₂ und erhitzt unter Rühren langsam auf 70 °C. Es wird bei dieser Temperatur 15 h nachgerührt. Nach Abkühlen erhält man eine dünnflüssige, weißlichblaue Suspension mit einem Gehalt an Penflufen von 1,44 % (HPLC).

Mittlere Teilchengröße (gemessen mit Laserbeugung) 0,365 µm ; 90 % der Teilchen (Vol%) sind kleiner 0,881 µm.

### Beispiel 5

Eine Lösung von 2,88 g SDS (Na-Dodecylsulfat), 0,163 g Natriumhydrogencarbonat in 200g Wasser wird mit einer organischen Lösung bestehend aus 91,7 g Methylmethacrylat, 4,3 g Penflufen, 3,84 g Hexadecan und 1,63 g 2,2'-Azobis(2-methylbutyronitril) versetzt und zum Erzeugen einer Emulsion 30 min mit einem Durchlaufultraturrax (IKA T 25 digital / Aufsatz DK 25.11; 6600 U/min) behandelt. Anschließend überführt man in einen 1000 ml Dreihalskolben, wäscht mit N₂ und erhitzt unter Rühren langsam auf 70 °C. Es wird bei dieser Temperatur 15 h nachgerührt. Nach dem Abkühlen erhält man eine dünnflüssige, weißliche Suspension mit einem Gehalt an Penflufen von 1,48 % (HPLC).

Mittlere Teilchengröße (gemessen mit Laserbeugung) 0,574 µm; 90 % der Teilchen (Vol%) sind kleiner 1,290 µm.

### Beispiel 6

Eine Lösung von 2,88 g SDS (Na-Dodecylsulfat), 0,163 g Natriumhydrogencarbonat in 200g Wasser wird mit einer organischen Lösung bestehend aus 91,7 g Methylmethacrylat, 4,3 g Penflufen, 3,84 g Hexadecan und 1,63 g 2,2'-Azobis(2-methylbutyronitril) versetzt und zum Erzeugen einer Emulsion 30 min durch einen Durchlaufultraturrax (IKA T 25 digital / Aufsatz DK 25.11; 14000 U/min) behandelt. Anschließend überführt man in einen 1000 ml Dreihalskolben, wäscht mit N₂ und erhitzt unter Rühren langsam auf 70 °C. Es wird bei dieser Temperatur 15 h nachgerührt. Nach dem Abkühlen erhält man eine dünnflüssige, weißliche Suspension mit einem Gehalt an Penflufen von 1,49 % (HPLC).

Mittlere Teilchengröße (gemessen mit Laserbeugung) 0,151 µm ; 90 % der Teilchen (Vol%) sind kleiner 0,364 µm.

### Vergleichsversuch 1 (mit wasserlöslichem radikalischem Initiator)

Eine Lösung von 1,15 g SDS (Na-Dodecylsulfat), 0,163 g Natriumhydrogencarbonat und 1,63 g Natriumperoxodisulfat in 144 g Wasser wird mit einer organischen Lösung bestehend aus 87,4 g Methylmethacrylat und 8,6 g Penflufen versetzt und zum Erzeugen einer Emulsion 30 min mit einem Ultraturrax (11000 U/min) behandelt. Anschließend überführt man in einen 1000 ml Dreihalskolben, wäscht mit N₂ und erhitzt unter Rühren langsam auf 70 °C. Nach ca 4 h verdickte sich die entstehenden Suspension und Penflufen kristallisierte aus. Unterdem Mikroskop sieht man eine Mischung sehr grober Partikel aus verklebtem Polymer und auskristallisiertem Penflufen.

Ein unter identischen Bedingungen durchgeführter Versuch mit AIBN (Azobis(isobutyronitril)), einem öllöslichen radikalischen Iniator führt zu einer feinteiligen Suspension ohne Auskristallisation von Penflufen.

## Patentansprüche

1. Penflufenhaltige Polymerpartikel, die durch Polymerisation mindestens eines ethylenisch, ungesättigten Monomeren in Gegenwart von Penflufen oder dessen Salze und Säureadditionsverbindungen und in Gegenwart eines öllöslichen radikalischen Initiators in einer Öl-in-Wasser Emulsionspolymerisation hergestellt werden, **dadurch gekennzeichnet, dass** als ein ethylenisch ungesättigter Monomer ein neutraler, ungeladener, ethylenisch ungesättigter Monomer des Typs M1 ausgewählt aus der Gruppe Styrol, Divinylbenzol, Ester monoethylenisch ungesättigter Mono- und Dicarbonsäuren mit 3 bis 8 C-Atomen und insbesondere 3 oder 4 C-Atomen mit C₁-C₂₀-Alkanolen oder mit C₅-C₈-Cycloalkanolen in einer Menge von mindestens 60 Gew. %, bezogen auf die Gesamtmenge der eingesetzten Monomeren, eingesetzt wird.

2. Penflufenhaltige Polymerpartikel gemäß Anspruch 1, **dadurch gekennzeichnet, dass** als ethylenisch, ungesättigte Monomeren, neutrale, ungeladene, ethylenisch ungesätigte Monomere des Typs M1 ausgewählt aus der Gruppe Methylacrylat, Ethylacrylat, n-Butylacrylat, Isobutylacrylat, tert.-Butylacrylat, n-Hexylacrylat, 2-Ethylhexylacrylat, 3-Propylheptylacrylat, Stearylacrylat Methylmethacrylat, Ethylmethacrylat, n-Butylmethacrylat, Isobutylmethacrylat, tert.-Butylmethacrylat, n-Hexylmethacrylat und Stearylmethacrylat, Divinylbenzol und Mischungen dieser Monomeren, eingesetzt werden.

3. Penflufenhaltige Polymerpartikel gemäß Ansprüchen 1 oder 2, **dadurch gekennzeichnet, dass** als ethylenisch, ungesättigte Monomeren, zusätzlich zu den Monomeren des Typs M1 ethylenisch, ungesätigte Monomere mit anionischen oder kationischen Gruppen des Typs M2 ausgewählt aus der Gruppe α,β-ethylenisch ungesättigte C₃-C₈-Mono- und C₄-C₈-Dicarbonsäuren, insbesondere Acrylsäure, Methacrylsäure, Crotonsäure, Maleinsäure, Fumarsäure und Itaconsäure, 2-(N,N,N-Trimethylammonium)ethylacrylat-Chlorid, 2-(N,N,N-Trimethylammonium)ethylmethacrylat-Chlorld, 2-(N,N,N-Trimethylammonium)-ethylmethacrylamid-Chlorid, 3-(N,N,N-Trimethylammonium)propylacrylamid-Chlorid, 3-(N,N,N-Trimethylammonium) propylmethacrylamid-Chlorid, 2-(N,N,N-Trimethylammonium)-ethylacrylamid-Chlorid, sowie die entsprechenden Methosulfate und Sulfate, eingesetzt werden.

4. Penflufenhaltige Polymerpartikel gemäß mindestens einem der Ansprüche 1 bis 3, dass die ethylenisch, ungesätigten Monomere zusammengesetzt sind aus 80 bis 99.9 Gew. % Monomere M1 und 0,1 Gew.% bis 20 Gew. % Monomere M2, bezogen auf die Gesamtmenge der eingesetzten Monomeren, enthalten.

5. Penflufenhaltige Polymerpartikel gemäß einem oder mehreren der Ansprüche 1 bis 4, **dadurch gekennzeichnet, dass** die öllöslichen Initiatoren ausgewählt sind aus der Gruppe 2,2'-Azobis-isobutyronitril, 2,2'-Azobis(2-methylbutyronitril), 2,2'-Azobis[2-methyl-N-(-2-hydroxyethyl)propionamid, 1,1'-Azobis(1-cyclo hexancarbonitril), 2,2'-Azobis(2,4-dimethylvaleronitril), 2,2'-Azobis(N,N'-dimethylenisobutyroamidin)dihydrochlorid, 2,2'-Azobis(2-amidinopropan) dihydrochlorid, Diacetylperoxid, Di-tert.-butylperoxid, Diamylperoxid, Dioctano-ylperoxid, Didecanoylperoxid, Dilauroylperoxid, Dibenzoylperoxid, Bis(o-toluyl)peroxid, Succinylperoxid, tert.-Butylperacetat, tert.-Butylpermaleinat, tert.-Butylperisobutyrat, tert.-Butylperpivalat, tert.-Butylperoctoat, tert.-Butylperneodecanoat, tert.-Butylperbenzoat, tert.-Butylperoxid, tert.-Butylhydroperoxid, Cumolhydroperoxid, tert.-Butylperoxi-2-ethylhexanoat und Diisopropylperoxidicarbamat und Mischungen dieser Verbindungen.

6. Penflufenhaltige Polymerpartikel gemäß einem oder mehreren der Ansprüche 1 bis 5, **dadurch gekennzeichnet, dass** die Menge an Penflufen oder dessen Salze und Säureadditionsverbindungen zwischen 0,5 bis 15 Gew.% bezogen auf die Gesamtmenge der eingesetzten Monomeren beträgt.

7. Penflufenhaltige Polymerpartikel gemäß einem oder mehreren der Ansprüche 1 bis 6, **dadurch gekennzeichnet, dass** zusätzlich weitere antimikrobiell wirksame Verbindungen, wie Fungizide, Bakterizide, Herbizide oder Insektizide enthalten sind.

8. Penflufenhaltige Polymerpartikel gemäß einem oder mehreren der Ansprüche 1 bis 7, **dadurch gekennzeichnet** ist, das die Partikel einen Durchmesser von 100 nm bis 1 µm haben.

9. Penflufenhaltige Mittel enthaltend penflufenhaltige Polymerpartikel gemäß einem oder mehreren der Ansprüche 1 bis 8, enthaltend mindestens ein Lösungs- oder Verdünnungsmittel.

10. Verfahren zur Herstellung von penflufenhaltigen Polymerpartikeln aus Anspruch 1, **dadurch gekennzeichnet, dass** als ein ethylenisch ungesättigter Monomeren ein neutraler, ungeladener, ethylenisch ungesättigter Monomeren des Typs M1 ausgewählt aus der Gruppe Styrol, Divinylbenzol, Ester monoethylenisch ungesättigter Mono- und Dicarbonsäuren mit 3 bis 8 und insbesondere 3 oder 4 C-Atomen mit C₁-C₂₀-Alkanolen oder mit C₅-C₈-Cycloalkanolen in einer Menge von mindestens 60 Gew. %, bezogen auf die Gesamtmenge der eingesetzten Monomeren, in Gegenwart von Penflufen oder dessen Salze und Säureadditionsverbindungen und in Gegenwart eines öllöslichen radikalischen Initiators in einer Öl-in-Wasser Emulsionspolymerisation polymerisiert wird.

11. Verfahren zur Herstellung von penflufenhaltigen Polymerpartikeln gemäß Anspruch 10, **dadurch gekennzeichnet, dass** die Polymerisation bei einer Temperatur von 50°C bis 90°C durchgeführt wird.

12. Verfahren zur Herstellung von penflufenhaltigen Polymerpartikeln gemäß Anspruch 10 oder 11, **dadurch gekennzeichnet, dass** als Costabilisatoren langkettige, lineare oder verzweigte oder cyklische, aliphatische Kohlenwasserstoffe ausgewählt aus der Gruppe C₁₄-C₂₅-Alkane und C₁₄-C₂₅-Cycloalkane oder deren Gemische eingesetzt werden.

13. Verwendung der penflufenhaltigen Polymerpartikel gemäß der Ansprüche 1 bis 8 und der penflufenhaltigen Mittel gemäß Anspruch 9 zum Schutz von technischen Materialien.

14. Verwendung gemäß Anspruch 13, **dadurch gekennzeichnet, dass** das technische Material Holz, Holz-Plastik-Kompositen und/oder ein Holzprodukt ist.

## Claims

1. Penflufen-containing polymer particles prepared by polymerization of at least one ethylenically unsaturated monomer in the presence of penflufen or its salts and acid addition compounds and in the presence of an oil-soluble radical initiator in an oil-in-water emulsion polymerization, **characterized in that** an ethylenically unsaturated monomer used is a neutral, uncharged, ethylenically unsaturated monomer of type M1 selected from the group consisting of styrene, divinylbenzene, esters of monoethylenically unsaturated monocarboxylic and dicarboxylic acids having 3 to 8 carbon atoms and more particularly 3 or 4 carbon atoms with C₁-C₂₀ alkanols or with C₅-C₈ cycloalkanols in an amount of at least 60 wt% based on the total amount of monomers used.

2. Penflufen-containing polymer particles according to Claim 1, **characterized in that** ethylenically unsaturated monomers used are neutral, uncharged, ethylenically unsaturated monomers of type M1 selected from the group consisting of methyl acrylate, ethyl acrylate, n-butyl acrylate, isobutyl acrylate, tert-butyl acrylate, n-hexyl acrylate, 2-ethylhexyl acrylate, 3-propylheptyl acrylate, stearyl acrylate, methyl methacrylate, ethyl methacrylate, n-butyl methacrylate, isobutyl methacrylate, tert-butyl methacrylate, n-hexyl methacrylate and stearyl methacrylate, divinylbenzene and mixtures of these monomers.

3. Penflufen-containing polymer particles according to Claim 1 or 2, **characterized in that** ethylenically unsaturated monomers used, in addition to the monomers of type M1, are ethylenically unsaturated monomers having anionic or cationic groups of type M2 selected from the group consisting of α,β-ethylenically unsaturated C₃-C₈ monocarboxylic and C₄-C₈ dicarboxylic acids, more particularly acrylic acid, methacrylic acid, crotonic acid, maleic acid, fumaric acid and itaconic acid, 2-(N,N,N-trimethylammonium)ethyl acrylate chloride, 2-(N,N,N-trimethylammonium)ethyl methacrylate chloride, 2-(N,N,N-trimethylammonium)ethylmethacrylamide chloride, 3-(N,N,N-trimethylammonium)propylacrylamide chloride, 3-(N,N,N-trimethylammonium)propylmethacrylamide chloride, 2-(N,N,N-trimethylammonium)ethylacrylamide chloride, and also the corresponding methosulfates and sulfates.

4. Penflufen-containing polymer particles according to at least one of Claims 1 to 3, that the ethylenically unsaturated monomers comprise are composed of 80 to 99.9 wt% of monomers M1 and 0.1 wt% to 20 wt% of monomers M2, based on the total amount of monomers used.

5. Penflufen-containing polymer particles according to one or more of Claims 1 to 4, **characterized in that** the oil-soluble initiators are selected from the group consisting of 2,2'-azobisisobutyronitrile, 2,2'-azobis(2-methylbutyronitrile), 2,2'-azobis[2-methyl-N-(2-hydroxyethyl)propionamide, 1,1'-azobis(1-cyclohexanecarbonitrile), 2,2'-azobis(2,4-dimethylvaleronitrile), 2,2'-azobis(N,N'-dimethyleneisobutyroamidine) dihydrochloride, 2,2'-azobis(2-amidinopropane) dihydrochloride, diacetyl peroxide, di-tert-butyl peroxide, diamyl peroxide, dioctanoyl peroxide, didecanoyl peroxide, dilauroyl peroxide, dibenzoyl peroxide, bis(o-tolyl) peroxide, succinyl peroxide, tert-butyl peracetate, tert-butyl permaleate, tert-butyl perisobutyrate, tert-butyl perpivalate, tert-butyl peroctoate, tert-butyl perneodecanoate, tert-butyl perbenzoate, tert-butyl peroxide, tert-butyl hydroperoxide, cumene hydroperoxide, tert-butyl peroxy-2-ethylhexanoate and diisopropyl peroxydicarbamate and mixtures of these compounds.

6. Penflufen-containing polymer particles according to one or more of Claims 1 to 5, **characterized in that** the amount of penflufen or its salts and acid addition compounds is between 0.5 to 15 wt%, based on the total amount of monomers used.

7. Penflufen-containing polymer particles according to one or more of Claims 1 to 6, **characterized in that** additionally further antimicrobially active compounds are present, such as fungicides, bactericides, herbicides or insecticides.

8. Penflufen-containing polymer particles according to one or more of Claims 1 to 7, is **characterized in that** the particles have a diameter of 100 nm to 1 µm.

9. Penflufen-containing compositions comprising penflufen-containing polymer particles according to one or more of Claims 1 to 8, comprising at least one solvent or diluent.

10. Process for preparing penflufen-containing polymer particles from Claim 1, **characterized in that** as an ethylenically unsaturated monomer a neutral, uncharged, ethylenically unsaturated monomer of type M1 selected from the group consisting of styrene, divinylbenzene, esters of monoethylenically unsaturated monocarboxylic and dicarboxylic acids having 3 to 8 and more particularly 3 or 4 carbon atoms with C₁-C₂₀ alkanols or with C₅-C₈ cycloalkanols in an amount of at least 60 wt%, based on the total amount of monomers used, is polymerized in the presence of penflufen or its salts and acid addition compounds and in the presence of an oil-soluble radical initiator in an oil-in-water emulsion polymerization.

11. Process for preparing penflufen-containing polymer particles according to Claim 10, **characterized in that** the polymerization is carried out at a temperature of 50°C to 90°C.

12. Process for preparing penflufen-containing polymer particles according to Claim 10 or 11, **characterized in that** costabilizers used are long-chain, linear or branched or cyclic, aliphatic hydrocarbons selected from the group consisting of C₁₄-C₂₅ alkanes and C₁₄-C₂₅ cycloalkanes or mixtures thereof.

13. Use of the penflufen-containing polymer particles according to Claims 1 to 8 and of the penflufen-containing compositions according to Claim 9 for protecting technical materials.

14. Use according to Claim 13, **characterized in that** the technical material is wood, wood-plastic composites and/or a wood product.

## Revendications

1. Particules polymères contenant du penflufène, qui sont fabriquées par polymérisation d'au moins un monomère éthyléniquement insaturé en présence de penflufène ou ses sels et composés d'addition acide et en présence d'un initiateur radicalaire soluble dans les huiles par une polymérisation en émulsion huile dans eau, **caractérisées en ce qu'**un monomère éthyléniquement insaturé neutre non chargé de type M1 choisi dans le groupe constitué par le styrène, le divinylbenzène, les esters d'acides mono- et dicarboxyliques monoéthyléniquement insaturés contenant 3 à 8 atomes C, et notamment 3 ou 4 atomes C, avec des alcanols en C₁-C₂₀ ou avec des cycloalcanols en C₅-C₈, est utilisé en tant que monomère éthyléniquement insaturé en une quantité d'au moins 60 % en poids, par rapport à la quantité totale des monomères utilisés.

2. Particules polymères contenant du penflufène selon la revendication 1, **caractérisées en ce que** des monomères éthyléniquement insaturés neutres non chargés de type M1 choisis dans le groupe constitué par l'acrylate de méthyle, l'acrylate d'éthyle, l'acrylate de n-butyle, l'acrylate d'isobutyle, l'acrylate de tert.-butyle, l'acrylate de n-hexyle, l'acrylate de 2-éthylhexyle, l'acrylate de 3-propylheptyle, l'acrylate de stéaryle, le méthacrylate de méthyle, le méthacrylate d'éthyle, le méthacrylate de n-butyle, le méthacrylate d'isobutyle, le méthacrylate de tert.-butyle, le méthacrylate de n-hexyle et le méthacrylate de stéaryle, le divinylbenzène et les mélanges de ces monomères sont utilisés en tant que monomères éthyléniquement insaturés.

3. Particules polymères contenant du penflufène selon la revendication 1 ou 2, **caractérisées en ce qu'**en plus des monomères de type M1, des monomères éthyléniquement insaturés contenant des groupes anioniques ou cationiques de type M2 choisis dans le groupe constitué par les acides monocarboxyliques en C₃-C₈ et dicarboxyliques en C₄-C₈ α,β-éthyléniquement insaturés, notamment l'acide acrylique, l'acide méthacrylique, l'acide crotonique, l'acide maléique, l'acide fumarique et l'acide itaconique, le chlorure d'acrylate de 2-(N,N-N-triméthylammonium)-éthyle, le chlorure de méthacrylate de 2-(N,N,N-triméthylammonium)-éthyle, le chlorure de 2-(N,N,N-triméthylammonium)-éthylméthacrylamide, le chlorure de 3-(N,N,N-triméthylammonium)-propylacrylamide, le chlorure de 3-(N,N,N-triméthylammonium)-propylméthacrylamide, le chlorure de 2-(N,N,N-triméthylammonium)-éthylacrylamide, ainsi que les méthosulfates et sulfates correspondants, sont utilisés en tant que monomères éthyléniquement insaturés.

4. Particules polymères contenant du penflufène selon au moins l'une quelconque des revendications 1 à 3, que les monomères éthyléniquement insaturés contiennent sont composés par 80 à 99,9 % en poids de monomères M1 et 0,1 % en poids à 20 % en poids de monomères M2, par rapport à la quantité totale des monomères utilisés.

5. Particules polymères contenant du penflufène selon une ou plusieurs des revendications 1 à 4, **caractérisées en ce que** les initiateurs solubles dans les huiles sont choisis dans le groupe constitué par le 2,2'-azobis-isobutyronitrile, le 2,2'-azobis(2-méthylbutyronitrile), le 2,2'-azobis[2-méthyl-N-(-2-hydroxyéthyl)propionamide, le 1,1'-azobis(1-cyclohexanecarbonitrile), le 2,2'-azobis(2,4-diméthylvaléronitrile), le dichlorhydrate de 2,2'-azobis(N,N'-diméthylène-isobutyroamidine), le dichlorhydrate de 2,2'-azobis(2-amidinopropane), le peroxyde de diacétyle, le peroxyde de di-tert.-butyle, le peroxyde de diamyle, le peroxyde de dioctanoyle, le peroxyde de didécanoyle, le peroxyde de dilauroyle, le peroxyde de dibenzoyle, le peroxyde de bis(o-toluyle), le peroxyde de succinyle, le peracétate de tert.-butyle, le permaléinate de tert.-butyle, le perisobutyrate de tert.-butyle, le perpivalate de tert.-butyle, le peroctoate de tert.-butyle, le pernéodécanoate de tert.-butyle, le perbenzoate de tert.-butyle, le peroxyde de tert.-butyle, l'hydroperoxyde de tert.-butyle, l'hydroperoxyde de cumène, le peroxy-2-éthylhexanoate de tert.-butyle et le peroxydicarbamate de diisopropyle et les mélanges de ces composés.

6. Particules polymères contenant du penflufène selon une ou plusieurs des revendications 1 à 5, **caractérisées en ce que** la quantité de penflufène ou ses sels et composés d'addition acide est comprise entre 0,5 et 15 % en poids, par rapport à la quantité totale des monomères utilisés.

7. Particules polymères contenant du penflufène selon une ou plusieurs des revendications 1 à 6, **caractérisées en ce que** des composés à activité antimicrobienne supplémentaires, tels que des fongicides, des bactéricides, des herbicides ou des insecticides, sont en outre contenus.

8. Particules polymères contenant du penflufène selon une ou plusieurs des revendications 1 à 7, est **caractérisées en ce que** les particules ont un diamètre de 100 nm à 1 µm.

9. Agents contenant du penflufène, contenant des particules polymères contenant du penflufène selon une ou plusieurs des revendications 1 à 8, contenant au moins un solvant ou un diluant.

10. Procédé de fabrication de particules polymères contenant du penflufène selon la revendication 1, **caractérisé en ce qu'**un monomère éthyléniquement insaturé neutre non chargé de type M1 choisi dans le groupe constitué par le styrène, le divinylbenzène, les esters d'acides mono- et dicarboxyliques monoéthyléniquement insaturés contenant 3 à 8 atomes C, et notamment 3 ou 4 atomes C, avec des alcanols en C₁-C₂₀ ou avec des cycloalcanols en C₅-C₈, est polymérisé en tant que monomère éthyléniquement insaturé en une quantité d'au moins 60 % en poids, par rapport à la quantité totale des monomères utilisés, en présence de penflufène ou ses sels et composés d'addition acide et en présence d'un initiateur radicalaire soluble dans les huiles, par une polymérisation en émulsion huile dans eau.

11. Procédé de fabrication de particules polymères contenant du penflufène selon la revendication 10, **caractérisé en ce que** la polymérisation est réalisée à une température de 50 °C à 90 °C.

12. Procédé de fabrication de particules polymères contenant du penflufène selon la revendication 10 ou 11, **caractérisé en ce que** des hydrocarbures aliphatiques linéaires ou ramifiés ou cycliques à chaîne longue choisis dans le groupe constitué par les alcanes en C₁₄-C₂₅ et les cycloalcanes en C₁₄-C₂₅ ou leurs mélanges sont utilisés en tant que co-stabilisateurs.

13. Utilisation des particules polymères contenant du penflufène selon les revendications 1 à 8 et des agents contenant du penflufène selon la revendication 9 pour la protection de matériaux techniques.

14. Utilisation selon la revendication 13, **caractérisée en ce que** le matériau technique est le bois, des composites bois-plastique et/ou un produit à base de bois.
